Europäisches Patentamt

European Patent Office

Office européen des brevets

(11) Numéro de publication: **0 302 781 B1**

# FASCICULE DE BREVET EUROPEEN

(45) Date de publication de fascicule du brevet: **11.11.92**

(51) Int. Cl.5: **A61K 39/085**, A61K 31/715, C12P 19/04

(21) Numéro de dépôt: **88401996.9**

(22) Date de dépôt: **29.07.88**

(54) **Procédé d'obtention de polyosides capsulaires de staphylocoques, applications de ces polyosides et souches pour la mise en oeuvre du procédé.**

(30) Priorité: **03.08.87 FR 8711006**

(43) Date de publication de la demande:
**08.02.89 Bulletin 89/06**

(45) Mention de la délivrance du brevet:
**11.11.92 Bulletin 92/46**

(84) Etats contractants désignés:
**AT BE CH DE ES FR GB GR IT LI LU NL SE**

(56) Documents cités:
**FR-A- 2 410 043**

**ANNALES INSTITUT PASTEUR, vol. 123, 1972, pages 783-797; J. FLEURETTE et al.: "Caractères biochimiques et antigéniques de 'Staphylococcus epidermidis'"**

**CHEMICAL ABSTRACTS, vol. 101, no. 11, 10 septembre 1984, page 458, résumé no. 88521h, Columbus, Ohio, US; J.M. FOURNIER et al.: "Purification and characterization of Staphylococcus sureus type 8 capsular polysaccharide", & INFECT. IMMUN. 1984, 45 (1), 87-93**

(73) Titulaire: **INSTITUT PASTEUR**
**28, rue du Docteur Roux**
**F-75724 Paris Cédex 15(FR)**

(72) Inventeur: **Fournier, Jean-Michel**
**100, rue de la Chapelle**
**F-75018 PARIS(FR)**
Inventeur: **Bouvet, Anne**
**203, rue d'Alésia**
**F-75014 PARIS(FR)**
Inventeur: **Boutonnier, Alain**
**151, rue du Château des Rentiers**
**F-75013 PARIS(FR)**

(74) Mandataire: **Le Guen, Gérard et al**
**CABINET LAVOIX 2, place d'Estienne d'Orves**
**F-75441 Paris Cédex 09(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

CHEMICAL ABSTRACTS, vol. 83, no. 7, 18 août 1975, page 328, résumé no. 56579b, Columbus, Ohio, US; G.S. JOHNSEN et al.: "Polysaccharide C of Staphylococcus epidermidis 2. Antigenic properties", & ACTA PATHOL. MICROBIOL. SCAND. SECT. B: MICROBIOL. 1975, 83B(3), 235-9

CHEMICAL ABSTRACTS, vol. 101, no. 19, 5 novembre 1984, page 353, résumé no. 167029g, Columbus, Ohio, US; Y. ICHIMAN et al.: "The role of the capsule of Staphylococcus epidermidis in experimental infection and immunity", & SEI MARIANNA IKA DAIGAKU ZASSHI 1984, 12(1), 1-9

CHEMICAL ABSTRACTS, vol. 93, no. 11, 15 septembre 1980, page 371, résumé no. 110261p, Columbus, Ohio, US; A. UMEDA et al.: "Staphilococcus cell wall structure and the mode of formation", & NIPPON SAIKINGAKU ZASSHI 1980, 35(1), 129

**Description**

La présente invention concerne un procédé d'obtention de polyosides capsulaires de staphylocoques et plus particulièrement un procédé d'obtention de polyosides capsulaires caractéristiques de Staphylococcus aureus.

S. aureus produit un très grand nombre d'antigènes extra- et intra-cellulaires, parmi lesquels de nombreuses toxines et enzymes. L'implication de ces métabolites dans les intoxications alimentaires ou dans la constitution des abcès est bien établie. Par contre, aucune corrélation n'a pu être établie entre la présence de ces antigènes et les propriétés invasives de S. aureus, c'est-à-dire sa capacité à entraîner une septicémie.

Les polyosides capsulaires sont des facteurs de pathogénicité bien connus pour des bactéries telles que Streptococcus pneumoniae, Haemophilus influenzae et Neisseria meningitidis. C'est pourquoi de nombreuses tentatives d'isolement de souches de staphylocoques capsulés ont été réalisées. Quelques souches capsulées ont été décrites dans la littérature (souches Smith, M et T). Cependant, le fait que les souches de S. aureus isolées chez des malades apparaissent dépourvues de capsules à l'examen de colonies sur gélose, ou à l'examen microscopique des germes, a conduit la majorité des auteurs à conclure que cette bactérie est habituellement non capsulée.

Pourtant, dans des publications parues depuis 1970, Walter W. Karakawa a démontré, d'une part, l'existence de polyosides capsulaires dans plusieurs isolements cliniques de staphylocoques et, d'autre part, que des antigènes capsulaires protégeaient la bactérie de la phagocytose par les polynucléaires. En 1980, Walter Karakawa et Willie Vann (Capsular polysaccharides of Staphylococcus aureus. p. 285-293. In J.B. Robbins, J.C. Hill, and J.C. Sadoff (ed.). Seminars in infectious disease. vol. 4. Bacterial vaccines. Thieme Stratton. Inc. New York) démontraient par des critères essentiellement immunologiques, l'existence d'au moins 8 types capsulaires de S. aureus.

La purification et la caractérisation biochimique et immunologique du polyoside capsulaire de type 8 ont été réalisées en 1984 (J.M. Fournier et al, Infect. Immun. 45 : 87-93) et une méthode de typage capsulaire de S. aureus a été décrite en 1985 (W.W. Karakawa et al. J. Clin. Microbiol. 22 : 445-447).

Des études épidémiologiques réalisées sur un grand nombre de souches de S. aureus isolées de malades, ont montré que 70 à 80% de ces souches possédaient l'un ou l'autre des polyosides capsulaires 5 et 8 (par exemple R.D. Arbeit et al. Diagn. Microbiol. Infect. Dis. 2 : 85-91. 1984).

Des anticorps monoclonaux spécifiques des polyosides capsulaires 5 et 8 ont été préparés (H.K. Hochkeppel et al. J. Clin. Microbiol. 25 : 526-530. 1987, et M.J. Nellers et al. Infect. Immun. 49 : 14-18. 1985).

Karakawa et al., J. Clin. Microbiol., 1985, 22 : 445-447 a en outre décrit une méthode d'identification des polysaccharides capsulaires de type 1, 2, 3, 4, 5, 6, 7 et 8 de Staphylococcus aureus au moyen d'une réaction d'agglutination et d'immunoprécipitation au moyen d'antisérums monospécifiques.

A côté de Staphylococcus aureus qui est un staphylocoque coagulase positive, c est-à-dire qui produit une coagulase libre (voir Bergey's Manual), on connaît de nombreuses espèces de staphylocoques qui sont des staphylocoques coagulase négative, c'est-à-dire ne produisent pas de coagulase Libre. Ces espèces coagulase négative ont été classées par KLOOS et SCHLEIFER; (Int. J. Syst. Bacteriol: 25, p. 50-61; p. 62-79).

Parmi ces espèces coagulase négative, on peut citer :

Staphylococcus simulans
Staphylococcus xylosus
Staphylococcus cohnii
Staphylococcus saprophiticus
Staphylococcus haemolyticus
Staphylococcus warneri
Staphylococcus hominis
Staphylococcus epidermidis
Staphylococcus capitis

Ces staphylocoques coagulase négative sont habituellement non pathogènes pour l'homme et pour l'animal. Aucun polyoside capsulaire identique à ceux décrits chez S. aureus n'a été identifié dans les souches de staphylocoques coagulase négative. Au contraire, M.J. Nelles et al (déjà cité) ont montré que 3 souches de S. epidermidis ne produisent pas de polyoside capsulaire de type 5 ou de type 8.

Or, la Demanderesse a découvert que certaines souches de staphylocoques coagulase négative produisent des polyosides capsulaires identiques à ceux de Staphylococcus aureus et généralement en des quantités plus importantes que Staphylococcus aureus. La fréquence d'isolement de telles souches est environ 2% chez l'homme.

La présente invention a en conséquence pour objet un procédé d'obtention de polyosides capsulaires caractéristiques de Staphylococcus aureus, par culture de souches de staphylocoques, extraction du polyoside capsulaire et purification de ce polyoside capsulaire, caractérisé en ce qu'on utilise comme souche de staphylocoque une souche de staphylocoque coagulase négative qui a été sélectionnée parmi les souches productrices de ce polyoside.

La présente invention a également pour objet les applications de ces polyosides notamment un procède d'obtention d'un vaccin contre Staphylococcus aureus et de produits de diagnostic, ainsi que des souches isolées et sélectionnées productrices de polyosides capsulaires caractéristiques de Staphylococcus aureus.

La sélection d'une souche productrice d'un polyoside capsulaire caractéristique de Staphylococcus aureus peut être aisément réalisée

- soit à partir de la bactérie intacte, par agglutination d'une suspension bactérienne au moyen d'anticorps spécifiques des polyosides capsulaires de Staphylococcus aureus, notamment d'anticorps monoclonaux tels que ceux déjà décrits, utilisés en solution ou préalablement fixés par exemple sur un support tel que des particules de latex,
- soit par détection du polyoside capsulaire dans des extraits bactériens, obtenus, par exemple,
- par lavage des bactéries
  ou
- après lyse bactérienne, obtenue par autoclavage ou par utilisation d'une enzyme spécifique du staphylocoque telle que la lysostaphine.

Le polyoside capsulaire peut être mis en évidence dans ces extraits bactériens par des méthodes immunologiques ou par des méthodes physico-chimiques.

Parmi les méthodes immunologiques, on peut citer :

- l'immunoprécipitation en gel d'agarose (double diffusion),
- l'immunoélectrophorèse en fusée (voir en particulier J.M. Fournier et al et Hochkeppel et al déjà cités).

L'anticorps spécifique du polyoside capsulaire est inclus dans un gel d'agarose équilibré dans un tampon à pH 8,6 (pH auquel l'anticorps ne migre pas). L'extrait bactérien est déposé dans un puits creusé dans le gel puis soumis à l'action d'un champ électrique qui fait migrer le polyoside capsulaire éventuellement présent dans l'extrait. Au fur et à mesure de sa migration, le polyoside capsulaire se combine avec l'anticorps et forme un précipité dont le front se déplace jusqu'à ce qu'il n'y ait plus de polyoside capsulaire libre. Après séchage, fixation et coloration du gel, la mesure de la hauteur de la flèche observée permet de déterminer la quantité de polyoside capsulaire présent dans l'extrait bactérien.

- les réactions immunoenzymatiques de type ELISA.

Parmi les méthodes physico-chimiques, on peut citer des méthodes qui mettent en évidence la présence d'un ou de plusieurs constituants du polyoside capsulaire :

- chromatographie en phase gazeuse,
- chromatographie liquide sous haute pression,
- chromatographie d'échange d'ions,
- filtration sur gel,
- chromatographie d'affinité,
- dosages chimiques des sucres aminés.

A partir des souches sélectionnées, on obtient le polyoside capsulaire en trois étapes :

- culture des bactéries,
- extraction du polyoside capsulaire,
- purification du polyoside capsulaire.

### 1) Culture des bactéries :

Comme milieu de culture, on peut utiliser notamment le milieu Columbia (Difco), liquide ou solide (meilleure production de polyoside capsulaire par des bactéries cultivées sur milieu solide), avec une durée de culture d'une nuit à 37°C par exemple.

### 2 ) Extraction du polyoside capsulaire :

Deux procédés peuvent être employés :
- autoclavage,

- lyse des bactéries par la lysostaphine.

a) **Extraction par autoclavage :**

Les bactéries cultivées sur milieu solide sont mises en suspension dans de l'eau physiologique tamponnée (EPT) (5 ml pour une boîte de Petri de 9 cm de diamètre), puis autoclavées une heure à 121°C. Après centrifugation 20 minutes à 25000 g, le surnageant est gardé et le culot est à nouveau extrait dans les mêmes conditions. Après centrifugation 20 minutes à 25000 g, les deux surnageants sont regroupés, dialysés contre l'eau distillée et lyophilisés. On obtient ainsi l'extrait brut (EB).

Pour 100 boîtes de Petri, on obtient approximativement 10 g de culot bactérien humide et 2 g d'EB.

Les bactéries cultivées en milieu liquide sont tuées par addition de phénol et d'éthanol (1,5 %, 24 h à la température du laboratoire), puis récupérées par centrifugation 20 minutes à 25000 g ou ultrafiltration par exemple sur un filtre Durapore type HV 0,45 micromètre de Millipore. Le culot bactérien est remis en suspension dans de l'EPT (0,1 g de culot humide/ml d'EPT) puis extrait à l'autoclavage dans les conditions décrites précédemment.

b) **Extraction par la lysostaphine :**

Le culot bactérien est mis en suspension dans de l'EPT (0,5 g/ml) contenant 25 mg de lysostaphine par litre. La suspension est agitée pendant une nuit à 37°C, puis centrifugée 20 minutes à 25000 g. Le surnageant est dialysé contre l'eau distillée puis lyophilisé.

3) **Purification du polyoside capsulaire :**

Une méthode avantageuse de purification est la suivante :

a) **Traitement enzymatique de l'EB :**

L'EB est repris dans l'EPT (10 mg/ml), traité par la DNase et la RNase (0,1 mg/ml, 6 h à 37°C) puis la protéase (1 mg/ml, une nuit à 37°C). Le produit est ensuite dialysé contre l'eau distillée et lyophilisé. Deux g d'EB donnent approximativement 0,7 g d'extrait traité aux enzymes (ETE). Ce traitement permet d'éliminer la plus grande partie des protéines et des acides nucléiques.

b) **Chromatographie d'échange d'ions :**

L'ETE est repris dans un tampon acétate de sodium (0,05 M, pH 6) contenant du chlorure de sodium (0,1 M) puis passé sur un échangeur d'ions (par exemple de la DEAE-cellulose) équilibré avec le même tampon. La plus grande partie des protéines contenues dans l'ETE ne sont pas absorbées sur l'échangeur d'ions et sont donc éliminées par un lavage de l'échangeur d'ions avec le tampon d'équilibrage. Par contre, le polyoside capsulaire, l'acide teichoïque et les acides nucléiques, restent fixés sur l'échangeur d'ions.

Le polyoside capsulaire est élué en faisant passer sur l'échangeur d'ions un tampon contenant une concentration plus élevée de chlorure de sodium. A titre d'exemple, le polyoside capsulaire de type 5 est élué avec un tampon acétate de sodium (0,05 M, pH 6) contenant du chlorure de sodium à la concentration de 0,15 M. Pour éluer le polyoside capsulaire de type 8, la concentration de chlorure de sodium doit être de 0.18 M. Les éluats sont recueillis dans un collecteur de fractions, et les fractions qui contiennent du polyoside capsulaire (détecté par une réaction immunologique utilisant des anticorps spécifiques) sont regroupées, dialysées contre l'eau distillée puis lyophilisées.

On obtient approximativement 20 mg d'extrait purifié sur échangeur d'ions (EEI) à partir de 0,17 g d'ETE. Cette chromatographie d'échange d'ions permet d'éliminer la plus grande partie des protéines, des acides nucléiques et de l'acide teichoïque.

c) **Filtration sur gel :**

L'EEI est reconstitué dans une solution de chlorure de sodium (0,2 M), et filtré sur un gel de Sepharose 4B-CL. Les fractions contenant du polyoside capsulaire, détecté par réaction immunologique, sont regroupées, dialysées contre l'eau distillée et lyophilisées. Le produit lyophilisé constitue le **polyoside capsulaire purifié.**

Ce polyoside capsulaire est contaminé par moins de 1% de protéines, moins de 0,5% d'acides nucléiques et moins de 0,05% d'acide teichoïque. A partir de 20 mg d'EEI, on obtient approximativement 10 mg de polyoside capsulaire purifié. Finalement, à partir de 10 g de culot bactérien humide récolté sur 100 boîtes de Petri, on obtient approximativement 10 mg de polyoside capsulaire purifié.

A titre d'exemple on a isolé les souches suivantes de staphylocoque coagulase négative produisant du polyoside capsulaire identique à celui de Staphylococcus aureas.

Pour la sélection des souches productrices de polyoside capsulaires caractéristiques de Staphylococcus aureus, on a mis au contact de la suspension bactérienne, une quantité d'anticorps spécifiques de ces polyosides capsulaires. La détection des polyosides capsulaires obtenus après lyse bactérienne, a été faite par immunoélectrophorèse en fusée (technique décrite par J.M. FOURNIER déjà cité) ou par Elisa.

Chaque souche hautement productrice de polyosides capsulaires a été caractérisée par ces deux techniques.

| Numéro de la souche | 850206 | 860638 | 850071 | 850279 |
|---|---|---|---|---|
| Numéro de dépôt à la CNCM | I-685 | I-682 | I-684 | I-683 |
| Date de dépôt. | 30.07.87 | 30.07.87 | 30.07.87 | 30.07.87 |
| Morphologie | Cocci gram positif | Cocci gram positif | Cocci gram positif | Cocci gram positif |
| Catalase | + | + | + | + |
| Fermentation du glucose en milieu MEVAG | + | + | + | + |
| DNAse | - | - | - | - |
| Coagulase | - | - | - | - |
| Type capsulaire | 5 | 5 | 8 | 8 |

Ces souches présentent les caractéristiques d'identification biochimique par le système Api Staph suivantes :

| Numéro de la souche | 850206 | 860638 | 850071 | 850279 |
|---|---|---|---|---|
| D-Glucose | + | + | + | + |
| D-Fructose | + | + | + | + |
| D-Mannose | ± | + | + | + |
| Maltose | + | + | + | + |
| Lactose | - | + | + | + |
| D-Tréhalose | + | + | + | + |
| D-Mannitol | - | - | - | - |
| Xylitol | - | - | - | - |

7

| | | | | |
|---|---|---|---|---|
| D-Mélibiose | − | − | − | − |
| Nitrate de potassium | + | − | + | + |
| $\alpha$-naphtyl phosphate | − | − | − | − |
| pyruvate de sodium | + | − | + | − |
| Raffinose | − | − | − | − |
| Xylose | − | − | − | − |
| Saccharose | + | + | + | + |
| $\alpha$-méthyl-D-glucoside | − | − | − | − |
| N-acétyl-glucosamine | + | + | + | + |

8

| | | | | |
|---|---|---|---|---|
| Arginine | + | − | − | − |

| | | | | |
|---|---|---|---|---|
| Urée | − | + | − | − |

| | | | | |
|---|---|---|---|---|
| Espèce * | Staphylo- coccus haemoly- ticus | Staphylo- coccus hominis | Staphylo- coccus hominis | Staphylo- coccus hominis |
| | 1 | 2 | 2 | 2 |

\* Selon la classification de Kloos et Schleifer

L'antibiogramme de ces souches est le suivant :

| Numéro de la souche | 850206 | 860638 | 850071 | 850279 |
|---|---|---|---|---|
| Pénicilline G | R | S | S | R |
| Oxacilline | R | S | S | S |
| Tobramycine | R | S | S | S |

| | | | | |
|---|---|---|---|---|
| Gentamicine | R | S | S | S |
| Tétracycline | R | S | S | R |
| Erythromycine | R | R | S | S |
| Clindamycine | S | S | S | S |
| Pristinamycine | S | S | S | S |
| Triméthoprime + Sulfamides | R | S | S | S |
| Rifampicine | S | S | S | S |
| Acide fusidique | R | S | S | S |
| Vancomycine | S | S | S | S |

R = résistant

S = sensible

A titre d'exemple, la mise en oeuvre du procédé d'obtention des polyosides capsulaires extraits par autoclavage de $10^9$ bactéries a permis d'obtenir les quantités indiquées dans le Tableau ci-après. Ces valeurs ne sont qu'indicatives, car la production de polyoside capsulaire est assez variable d'une culture à l'autre.

| Numéro de la Souche | Espèce | Type Capsulaire | Production de polyoside capsulaire (microgrammes) |
|---|---|---|---|
| 850206 | S. haemolyticus 1 | 5 | 30 |
| 860638 | S. hominis 2 | 5 | 4 |
| 850071 | S. hominis 2 | 8 | 20 |
| 850279 | S. hominis 2 | 8 | 5 |

Les polyosides capsulaires obtenus à partir de souches de staphylocoque coagulase négative peuvent être utilisés pour :

a) la préparation de vaccins contre Staphylococcus aureus, à usage humain ou vétérinaire, en vue - d'une protection individuelle

ou - de l'obtention de sérums immuns utilisables pour la préparation d'anticorps anti-polyoside capsulaire pouvant être utilisé en immunothérapie passive ou pour le diagnostic par des techniques d'agglutination, ELISA, RIA ou Western blot selon des méthodes connues de l'homme de l'art;

b) la préparation de produits de diagnostic pour un diagnostic chez l'homme, chez l'animal ou dans des produits agro-alimentaire, et notamment pour :

- la détection dans des produits biologiques d'anticorps spécifiques du polyoside capsulaire, ou
- la détection de polyoside capsulaire dans des isolements cliniques de staphylocoques coagulase positive ou coagulase négative.

**Revendications**

1. Procédé d'obtention d'un polyoside capsulaire caractéristique de Staphylococcus aureus par culture de souches de staphylocoques, extraction du polyoside capsulaire et purification de ce polyoside capsulaire, caractérisé en ce qu'on utilise comme souche de staphylocoque une souche de staphylocoque coagulase négative qui a été sélectionnée parmi les souches productrices de ce polyoside.

2. Procédé selon la revendication 1, caractérisé en ce qu'on sélectionne une souche de staphylocoque coagulase négative productrice d'un polyoside capsulaire caractéristique de Staphylococcus aureus par réaction d'agglutination au moyen d'anticorps spécifiques des polyosides capsulaires de Staphylococcus aureus.

3. Procédé selon la revendication 1, caractérisé en ce qu'on sélectionne une souche de staphylocoque coagulase négative productrice d'un polyoside capsulaire présenté par une souche de Staphylococcus aureus par détection du polyoside capsulaire dans un extrait bactérien de la souche.

4. Procédé selon la revendication 3, caractérisé en ce que l'extrait bactérien est un extrait obtenu par lavage des bactéries.

5. Procédé selon la revendication 3, caractérisé en ce que l'extrait bactérien est un extrait obtenu après lyse bactérienne.

6. Procédé selon l'une quelconque des revendications 1 à 5, caractérisé en ce qu'on extrait le polyoside capsulaire après autoclavage.

7. Procédé selon l'une quelconque des revendications 1 à 5, caractérisé en ce qu'on extrait le polyoside capsulaire après lyse des bactéries par un agent de lyse.

8. Procédé selon l'une quelconque des revendications 1 à 7, caractérisé en ce qu'on purifie le polyoside capsulaire extrait par traitement enzymatique, suivi d'une chromatographie d'échange d'ions et d'une filtration sur gel.

9. Procédé selon l'une quelconque des revendications 1 à 8, caractérisé en ce qu'on utilise une souche de staphylocoque coagulase négative déposée à la CNCM sous les numéros I-682, I-683, I-684 ou I-685.

10. Procédé d'obtention d un vaccin contre Staphylococcus aureus, caractérisé en ce que l'on utilise un polyoside selon la revendication 1.

**11.** Procédé d'obtention d'un produit de diagnostic pour la détection des staphylocoques coagulase positive ou négative, caractérisé en ce que l'on utilise un polyoside obtenu par un procédé selon la revendication 1.

**12.** Souches purifiées de staphylocoque coagulase négative productrices de polyoside capsulaire caractéristique de Staphylococcus aureus.

**13.** Souches selon la revendication 12, qui sont les souches déposées à la CNCM sous les numéros I-682, I-683, I-684 et I-685, le 30 juillet 1987.

**14.** Utilisation des souches selon la revendication 12 ou la revendication 13 pour l'obtention de polyosides capsulaires caractéristiques de Staphylococcus aureus.

**Claims**

**1.** Process for the preparation of a capsular polysaccharide characteristic of Staphylococcus aureus, by cultivating strains of staphylococci, extracting the capsular polysaccharide and purifying this capsular polysaccharide, characterised in that the strain of staphylococci used is a strain of coagulase-negative staphylococci selected from the strains which produce this polysaccharide.

**2.** Process according to claim 1, characterised in that a coagulase-negative strain of staphylococci producing a capsular polysaccharide characteristic of Staphylococcus aureus is selected by an agglutination reaction using antibodies specific for the capsular polysaccharides of Staphylococcus aureus.

**3.** Process according to claim 1, characterised in that a coagulase-negative strain of staphylococci producing a capsular polysaccharide presented by a strain of Staphylococcus aureus is selected by detection of the capsular polysaccharide in a bacterial extract of the strain.

**4.** Process according to claim 3, characterised in that the bacterial extract is obtained by washing the bacteria.

**5.** Process according to claim 3, characterised in that the bacterial extract is obtained after bacterial lysis.

**6.** Process according to any one of claims 1 to 5, characterised in that the capsular polysaccharide is extracted after autoclaving.

**7.** Process according to any one of claims 1 to 5, characterised in that the capsular polysaccharide is extracted after lysis of the bacteria by a lytic agent.

**8.** Process according to any one of claims 1 to 7, characterised in that the extracted capsular polysaccharide is purified by enzymatic treatment, followed by ion exchange chromatography and gel filtration.

**9.** Process according to any one of claims 1 to 8, characterised in that a coagulase-negative strain of staphylococci deposited with the CNCM under the numbers I-682, I-683, I-684 or I-685 is used.

**10.** Process for preparing a vaccine agent Staphylococcus aureus, characterised in that a polysaccharide according to claim 1 is used.

**11.** Process for preparing a diagnostic product for detective coagulase -positive or -negative staphylococci, characterised in that a polysaccharide obtained by a process according to claim 1 is used.

**12.** Purified strains of coagulase-negative staphylococci producing capsular polysaccharide characteristic of Staphylococcus aureus.

**13.** Strains according to claim 12 which are the strains deposited with the CNCM, under the numbers I-682,

EP 0 302 781 B1

I-683, I-684 and I-685, on 30th July 1987.

**14.** Use of the strains according to claim 12 or 13 for the production of capsular polysaccharides characteristic of <u>Staphylococcus aureus.</u>

**Patentansprüche**

**1.** Verfahren zur Gewinnung eines für <u>Staphylococcus aureus</u> charakteristischen Kapselpolyosids durch Züchten von Staphylokokkenstämmen, Extrahieren des Kapselpolyosids und Reinigen dieses Kapselpolyosids, **dadurch gekennzeichnet,** daß man als Staphylokokkenstamm einen Koagulase-negativen Staphylokokkenstamm verwendet, der aus dieses Polyosid bildenden Stämmen ausgewählt worden ist.

**2.** Verfahren nach Anspruch 1, **dadurch gekennzeichnet,** daß man einen Koagulase-negativen Staphylokokkenstamm, der ein für <u>Staphylococcus aureus</u> charakteristisches Kapselpolyosid bildet, durch Agglutinationsreaktion mit Hilfe von für Kapselpolyoside von <u>Staphylococcus aureus</u> spezifischen Antikörpern auswählt.

**3.** Verfahren nach Anspruch 1, **dadurch gekennzeichnet,** daß man einen Koagulase-negativen Staphylokokkenstamm, der ein Kapselpolyosid bildet, das von einem Stamm von <u>Staphylococcus aureus</u> gebildet wird, durch Nachweis des Kapselpolyosids in einem Bakterienextrakt des Stammes auswählt.

**4.** Verfahren nach Anspruch 3, **dadurch gekennzeichnet,** daß der Bakterienextrakt ein durch Waschen der Bakterien erhaltener Extrakt ist.

**5.** Verfahren nach Anspruch 3, **dadurch gekennzeichnet,** daß der Bakterienextrakt ein durch Lyse der Bakterien erhaltener Extrakt ist.

**6.** Verfahren nach einem der Ansprüche 1 bis 5, **dadurch gekennzeichnet,** daß man das Kapselpolyosid nach der Behandlung im Autoklaven extrahiert.

**7.** Verfahren nach einem der Ansprüche 1 bis 5, **dadurch gekennzeichnet,** daß man das Kapselpolyosid nach der Lyse der Bakterien mit einem Lysemittel extrahiert.

**8.** Verfahren nach einem der Ansprüche 1 bis 7, **dadurch gekennzeichnet,** daß man das extrahierte Kapselpolyosid durch enzymatische Behandlung, gefolgt von einer Ionenaustauschchromatographie und einer Gelfiltration, reinigt.

**9.** Verfahren nach einem der Ansprüche 1 bis 8, **dadurch gekennzeichnet,** daß man einen Koagulase-negativen Staphylokokkenstamm verwendet, der unter den Hinterlegungsnummern I-682, I-683, I-684 oder I-685 beim CNCM hinterlegt worden ist.

**10.** Verfahren zur Herstellung eines Impfstoffs gegen <u>Staphylococcus aureus</u>, **dadurch gekennzeichne**t, daß man ein Polyosid nach Anspruch 1 verwendet.

**11.** Verfahren zur Herstellung eines diagnostischen Produkts zum Nachweis von Koagulase-positiven oder -negativen Staphylokokken, **dadurch gekennzeichnet,** daß man ein nach einem Verfahren gemäß Anspruch 1 erhaltenes Polyosid verwendet.

**12.** Gereinigte, Koagulase-negative Staphylokokkenstämme, die das für <u>Staphylococcus aureus</u> charakteristische Kapselpolyosid bilden.

**13.** Stämme nach Anspruch 12, bei denen es sich um die Stämme handelt, die unter den Hinterlegungsnummern I-682-, I-683, I-684 und I-685 am 30.Juli 1987 beim CNCM hinterlegt worden sind.

**14.** Verwendung der Stämme nach Anspruch 12 oder nach Anspruch 13 zur Gewinnung von für <u>Staphylococcus aureus</u> charakteristischen Kapselpolyosiden.

13